Europäisches Patentamt

European Patent Office

Office européen des brevets

(11) Veröffentlichungsnummer: **0 503 374 A2**

## EUROPÄISCHE PATENTANMELDUNG

(21) Anmeldenummer: **92103300.7**

(22) Anmeldetag: **26.02.92**

(51) Int. Cl.5: **B01D 17/12**, B01D 17/04

(30) Priorität: **09.03.91 DE 4107643**

(43) Veröffentlichungstag der Anmeldung:
**16.09.92 Patentblatt 92/38**

(84) Benannte Vertragsstaaten:
**AT BE CH DE ES FR GB IT LI NL**

(71) Anmelder: **BAYER AG**

**W-5090 Leverkusen 1 Bayerwerk(DE)**

(72) Erfinder: **Augustin, Thomas, Dr.**
**Amsterdamer Strasse 70**
**W-5000 Köln 60(DE)**
Erfinder: **Keldenich, Jörg, Dr.**
**Heidackerstrasse 30**
**W-4018 Langenfeld(DE)**

(54) **Verfahren zur Spaltung von Öl-in-Wasser-Emulsionen.**

(57) Optimale Spaltmittelmengen zur Spaltung von niedrigsalinären und hochtensidhaltigen Öl-in-Wasser-Emulsionen werden ermittelt, in dem man das Strömungspotential in der zu spaltenden Emulsion mißt.

EP 0 503 374 A2

Die vorliegende Erfindung betrifft ein besonders vorteilhaftes Verfahren zur Spaltung bestimmter Öl-in-Wasser-Emulsionen.

Bei der Metallbe- und Metallverarbeitung, der Altölaufbereitung und anderen Gelegenheiten fallen niedrigsalinäre und hochtensidhaltige Öl-in-Wasser-Emulsionen an. Aus ökonomischen und ökologischen Gründen werden solche Emulsionen aufgearbeitet, indem man einen oder mehrere Emulsionsspalter hinzufügt, so eine möglichst reine wässrige und eine organische Phase erhält, die wässrige Phase, gegebenenfalls nach weiteren Behandlungsmaßnahmen über Vorfluter entsorgt und die organische Phase, gegebenenfalls auch nach weiteren Behandlungsmaßnahmen, aufarbeitet oder verbrennt.

Um bei der Emulsionsspaltung gute Ergebnisse zu erhalten ist es erforderlich, daß jeweils eingesetzte Spaltmittel genau zu dosieren. Eine Unterdosierung des Spaltmittels ergibt keine reine wässrige, d.h. weitgehend ölfreie Phase und/oder keine reine organische, d.h. weitgehend wasserfreie Phase. Eine Überdosierung des Spaltmittels ergibt zwar häufig zunächst eine Phasentrennung, die durch Reemulgierung jedoch wieder erheblich verschlechtert wird.

Bislang ist bekannt geworden, daß man mit optischen Systemen den Kohlenwasserstoffgehalt von wässrigen Phasen bestimmen kann (siehe z.B. US-PS 3 899 688, US-PS 4 128 833, EP-OS 256 431 und DE-OS 3 712 106). Nachteilig bei derartigen optischen Systemen ist die Empfindlichkeit der zu verwendenden Sensoren und Detektoren gegen Verschmutzung, die zu verfälschten Meßergebnissen führt. Deshalb ist diese Meßmethode zur Ermittlung optimaler Spaltmittelmengen für die Spaltung von Öl-in-Wasser-Emulsionen nicht geeignet.

Bekannt zur Ermittlung optimaler Spaltmittelmengen ist auch der sog. JAR-Test. Hierbei wird an Versuchsreihen mit gleicher Zusammensetzung der zu spaltenden Emulsion und wechselnden Spaltmittelmengen rein visuell ermittelt, welche Spaltmittelmenge optimale Spaltergebnisse erbringt. Diese visuelle Methode beinhaltet ebenfalls ein hohes Fehlerrisiko, da kein physikalischer Parameter gemessen wird, sondern lediglich visuelle Abschätzungen vorgenommen werden. Außerdem ist diese Methode sehr zeitaufwendig, sie kann nicht für eine kontinuierliche Ermittlung der optimalen Spaltmittelmenge angewendet werden und mit ihr kann man nicht die Spaltmittelmenge an kurzfristig eintretende Veränderungen in der Zusammensetzung der zu spaltenden Emulsion anpassen.

Aus der US-PS 4 947 885 ist bekannt, daß man in hochsalinären Raffinerieabwässern, die sehr geringe Mengen an Kohlenwasserstoffen und keine Tenside enthalten, den Gehalt an Kohlenwasserstoff bestimmen kann, indem man die elektrische Ladung von Öltröpfchen in den salzhaltigen Abwässern mißt. Es konnte nicht erwartet werden, daß diese Methode auf andere flüssig-flüssig-Systeme, insbesondere tensidenthaltende Systeme mit niedrigen Salz- und hohem Öl-Gehalt anwendbar sein könnte.

Es wurde nun ein Verfahren zur Spaltung von niedrigsalinären und hochtensidhaltigen Öl-in-Wasser-Emulsionen mit Spaltmitteln gefunden, das dadurch gekennzeichnet ist, daß man die jeweils optimale Spaltmittelmenge ermittelt durch Messung des Strömungspotentials in der zu spaltenden Emulsion.

Niedrigsalinäre Emulsionen im Sinne der vorliegenden Erfindung sind solche, z.B. weniger als 3,0 Gew.-%, vorzugsweise weniger als 2,5 Gew.-% Salze enthalten.

Hochtensidhaltige Emulsionen im Sinne der vorliegenden Erfindung sind solche, z.B. mehr als 0,1 Gew.-%, vorzugsweise 0,5 bis 5,0 Gew.-% Tenside enthalten. Bei den Tensiden kann es sich beispielsweise um anionische, kationische oder nichtionische Tenside handeln.

Der Ölgehalt der in das erfindungsgemäße Verfahren einzusetzenden Öl-in-Wasser-Emulsionen kann beispielsweise 0,5 bis 5 Gew.-% betragen, vorzugsweise liegt er bei 0,8 bis 2,5 Gew.-%.

In das erfindungsgemäße Verfahren einsetzbare Öl-in-Wasser-Emulionen können von verschiedener Herkunft sein. Beispielsweise kann es sich um mineralölbasische und teilsynthetische Kühlschmieremulsionen, Walzölemulsionen, sonstige Metallbe- und Metallverarbeitungshilfsmittel, um Flüssigkeiten aus der Altölaufbereitung und um ölhaltige Waschlaugen, Lackiererreiabwässer, Entfettungsbäder, ölhaltige Kondensate, Tankreinigungsabwässer, Bilgenwässer, slop-Öle und beliebige sonstige ölhaltige Abwässer handeln.

Im erfindungsgemäßen Verfahren können beliebige Spaltmittel verwendet werden. Als anorganische Spaltmittel kommen z.B. Salze von zwei- und dreiwertigen Metallen infrage, wie Calciumchlorid, Calciumoxid, Aluminiumchlorid, Aluminiumsulfat und Eisensulfat. Organische Emulsionsspalter können z.B. sein Polyamine, Polyamidamine, Polyimine, Polyetherpolyamine, quarternierte Polyamine, quarternierte Polyamidamine, Homo-, Co- und Terpolymere auf der Basis von Acrylsäure und Acrylamid, und Homo-, Co- und Terpolymerisate von Diallydimethylammoniumchlorid und Mischungen solcher Spalter.

Solche anorganischen und organischen Spaltmittel sind an sich bekannt.

Es ist das wesentliche Merkmal der vorliegenden Erfindung, die optimale Spaltmittelmenge durch Messung des Strömungspotentials der zu spaltenden Emulsion zu ermitteln. Man kann dabei so verfahren, daß man eine Probe der zu spaltenden Emulsion in ein Gerät zur Messung des Strömungspotentials einbringt, dann langsam unter Vermischung das oder die jeweils gewünschte(n) Spaltmittel solange hinzufügt, bis das Strömungspotential möglichst nahe bei Null liegt oder Null beträgt. Aus der hierfür benötigten Spaltmittelmenge läßt sich dann die für die Gesamtmenge der zu spaltenden Emulsion benötigte optimale Spaltmittelmenge berechnen.

Man kann auch so verfahren, daß man aus einem größeren Behälter, in dem sich die zu spaltende Emulsion befindet, und in den das oder die jeweils gewünschte(n) Spaltmittel zugegeben werden, einen Seitenstrom durch ein Gerät zur Messung des Strömungspotentials leitet und die Zugabe an Spaltmittel dann abbricht, wenn das gemessene Potential möglichst nahe bei Null liegt oder Null beträgt. Diese Arbeitsweise kann auch automatisiert werden.

Schließlich ist es möglich, bei kontinuierlich anfallenden Öl-in-Wasser-Emulsionen, auch mit wechselnden Zusammensetzungen, im Haupt- oder einem Seitenstrom die jeweils optimale Spaltmittelmenge durch Bestimmung der Abweichung des Strömungspotentials von Null und Ermittlung der benötigten Spaltmittelmenge, um das Strömungspotential auf Null zu bringen, der Öl-in-Wasser-Emulsion laufend eine optimale Spaltmittelmenge zuzuführen.

Das erfindungsgemäße Verfahren kann beispielsweise bei Temperaturen im Bereich von 10 bis 90°C durchgeführt werden.

Für die Bestimmung des Strömungspotentials ist beispielsweise der Partikelladungsdetektor PCD 02 der Firma Mütek, Herrsching, geeignet. Für diesen ist bis jetzt nur bekannt, daß man damit Dispersionen, d.h. Feststoffe enthaltende Systeme oder Polyelektrolytlösungen untersuchen und titrieren kann.

Das erfindungsgemäße Verfahren hat den Vorteil, daß man damit an Öl-in-Wasser-Emulsionen die optimale Spaltmittelmenge auf einfache und reproduzierbare Weise auf der Basis einer physikalischen Messung bestimmen kann.

Beispiele

Beispiel 1

Eine mineralölhaltige verbrauchte Kühlschmieremulsion für die Bearbeitung von Metallen, die weniger als 1,0 Gew.-% Salz, 1,5 Gew.-% Tenside und 2,0 Gew.-% Öl enthielt, wurde mit verschiedenen organischen Emulsionsspaltern (jeweils 1 Gew.-%ige Lösung in Wasser) gespalten. Damit wurde jeweils einmal das Spaltmittel nach der visuellen Methode (JAR-Test) und einmal nach dem erfindungsgemäßen Verfahren unter zur Hilfenahme eines Partikelladungsdetektors PCD 02 der Firma Mütek, Herrsching, bestimmt. Danach wurden in der wässrigen Phase die üblichen integralen Abwasserparameter bestimmt, welche Rückschlüsse auf den Gehalt der wässrigen Phase an Kohlenwasserstoffen zulassen und zwar der Kohlenwasserstoffgehalt nach DIN 38 409 H18, der CSB-Wert nach DIN 38 409 H41 und der TOC-Wert nach DIN 38 409 H3.

Die ermittelten Ergebnisse sind aus Tabelle 1 ersichtlich.

## Tabelle 1

| Spaltmittel (Handelsprodukte auf der Basis von Polyamidamin) | Dosiermethode a = visuell b = erfindungsgemäß | Kohlenwasserstoffgehalt (mg/l) | CSB-Wert (mg/l) | TOC-Wert (mg/l) |
|---|---|---|---|---|
| Typ 1 | a | 135 | 11.580 | 3.300 |
|  | b | 120 | 11.800 | 3.340 |
| Typ 2 | a | 155 | 10.300 | 3.200 |
|  | b | 30 | 9.850 | 3.190 |
| Typ 3 | a | 125 | 11.300 | 3.280 |
|  | b | 10 | 8.500 | 3.175 |
| Typ 4 | a | 75 | 10.400 | 3.200 |
|  | b | 25 | 11.200 | 3.270 |
| Typ 5 | a | 125 | 10.600 | 3.190 |
|  | b | 20 | 10.200 | 3.140 |

Beispiel 2

Eine mineralölhaltige verbrauchte Kühlschmieremulsion aus einem Werkstattbetrieb mit einem Salzgehalt von weniger als 1,0 Gew.-%, einem Tensidgehalt von 1,0 Gew.-% und einem Ölgehalt von 1,5 Gew.-% wurde wie in Beispiel 1 beschrieben gespalten und anschließend die wässrige Phase untersucht. Die erhaltenen Ergebnisse sind aus Tabelle 2 ersichtlich.

**Tabelle 2**

| Spaltmittel (Handelsprodukt auf der Basis Polyamidamin) | Dosiermethode a = visuell b = erfindungsgemäß | Kohlenwasserstoffgehalt (mg/l) | CSB-Wert (mg/l) | TOC-Wert (mg/l) |
|---|---|---|---|---|
| Typ 1 | a | 550 | 25.400 | 5.990 |
|  | b | 410 | 15.100 | 4.700 |
| Typ 2 | a | 95 | 27.770 | 4.530 |
|  | b | 53 | 14.600 | 4.500 |
| Typ 5 | a | 175 | 23.200 | 4.600 |
|  | b | 40 | 14.500 | 4.500 |

**Patentansprüche**

1. Verfahren zur Spaltung von niedrigsalinären und hochtensidhaltigen Öl-in-Wasser-Emulsionen mit Spaltmitteln, dadurch gekennzeichnet, daß man die jeweils optimale Spaltmittelmenge ermittelt durch Messung des Strömungspotentials in der zu spaltenden Emulsion.

2. Verfahren nach Anspruch 1, dadurch gekennzeichnet, daß die zu spaltende Emulsion weniger als 3,0 Gew.-% Salze und mehr als 0,1 Gew.-% Tenside enthält.

3. Verfahren nach Ansprüchen 1 und 2, dadurch gekennzeichnet, daß die zu spaltende Emulsion weniger als 2,5 Gew.-% Salze und 0,5 bis 5,0 Gew.-% Tenside enthält.

4. Verfahren nach Ansprüchen 1 bis 3, dadurch gekennzeichnet, daß die zu spaltende Emulsion 0,5 bis 5 Gew.-% Öl enthält.

5. Verfahren nach Ansprüchen 1 bis 4, dadurch gekennzeichnet, daß man eine Probe der zu spaltenden Emulsion in ein Gerät zur Messung des Strömungspotentials einbringt, dann langsam unter Vermischung das gewünschte Spaltmittel hinzufügt, bis das Strömungspotential möglichst nahe bei Null liegt oder Null beträgt und aus der hierfür benötigten Spaltmittelmenge die für die Gesamtmenge der zu spaltenden Emulsion benötigte optimale Spaltmittelmenge berechnet.

6. Verfahren nach Ansprüchen 1 bis 4, dadurch gekennzeichnet, daß man aus einem Behälter, in dem sich die zu spaltende Emulsion befindet und in den das gewünschte Spaltmittel zugegeben wird, einen Seitenstrom durch ein Gerät zur Messung des Strömungspotential leitet und die Zugabe an Spaltmittel dann abbricht, wenn das gemessene Potential möglichst nahe bei Null liegt oder Null beträgt.

7. Verfahren nach Ansprüchen 1 bis 4, dadurch gekennzeichnet, daß man bei kontinuierlich anfallenden Öl-in-Wasser-Emulsionen die Abweichung des Strömungspotentials von Null bestimmt, die Spaltmittelmenge ermittelt, die benötigt wird um das Strömungspotential auf Null zu bringen und der Öl-in-Wasser-Emulsion laufend die so ermittelte optimale Spaltmittelmenge zuführt.

8. Verfahren nach Ansprüchen 1 bis 7, dadurch gekennzeichnet, daß man es bei Temperaturen im Bereich von 10 bis 90°C durchführt.

5